# NEW EUROPEAN PATENT SPECIFICATION

(11) **EP 0 276 541 B2**
(45) Date of publication and mention of the opposition decision: **26.08.1998**
(45) Mention of the grant of the patent: 24.03.1993
(21) Application number: 87308690.4
(22) Date of filing: 30.09.1987
(51) Int. Cl.: C12P 7/64

(54) **Process for production of arachidonic acid**
Verfahren zur Herstellung von Arachidonsäure
Procédé de préparation de l'acide arachidonique

(30) Priority: 28.01.1987 JP 15920/87
(43) Date of publication of application: 03.08.1988
(73) Proprietor: SUNTORY LIMITED, Kita-ku, Osaka-shi, Osaka-fu 530 (JP)
(72) Inventor: Shinmen, Yoshifumi, Otokuni-gun Kyoto (JP); Yamada, Hideaki, Kyoto-shi Kyoto (JP); Shimizu, Sakayu, Kyoto-shi Kyoto (JP)
(74) Representative: Stoner, Gerard Patrick

(56) References cited:
- EP-A- 0 155 420
- EP-A- 0 207 475
- EP-A- 0 223 960
- CANADIAN JOURNAL OF MICROBIOLOGY, vol. 10, 1964, pages 187-195; R.H. HASKINS et al.: "Steroids and the stimulation of sexual reproduction of a species of phythium"
- AGRIC. BIOL. CHEM., vol. 51, no. 3, March 1987, pages 785-790; H. YAMADA et al.: "Production of arachidonic acid by mortierella elongata IS-5"
- CHEMICAL ABSTRACTS, vol. 105, no. 25, 22nd December 1986, page 625, abstract no. 224603y, Columbus, Ohio, US; & JP-A-61 177 990 (HARIMA CHEMICALS, INC.) 09-09-1986
- CHEMICAL ABSTRACTS, vol. 87, no. 19, 7th November 1977, page 433, abstract no. 150207h, Columbus, Ohio, US; & JP-A-77 64 484 (H. IIZUKA et al.) 27-05-1977
- CHEMICAL ABSTRACTS, vol. 101, no. 19, 5th November 1984, page 539, abstract no. 169107e, Columbus, Ohio, US; & JP-A-59 130 191 (AGENCY OF INDUSTRIAL SCIENCES AND TECHNOLOGY) 26-07-1984

## Description

The present invention relates to a new process for the production of arachidonic acid and a lipid comprising arachidonic acid.

Known processes for the production of arachidonic acid are those using microorganisms, i.e, Penicillium, Aspergillus, Rhodotorula or Fusarium, as disclosed in Japanese Examined Patent Publication Nos. 56-19231, 56-19232, and 56-19233. These processes, however, have the disadvantages of a low yield, long term fermentation, and a complicated production process.

However, a process for the production of arachidonic acid using a microorganism belonging to the genus Mortierella is not known.

In a first aspect this invention provides a process for production of arachidonic acid, comprising
culturing a microorganism, of the genus Mortierella and capable of producing arachidonic acid, in a culture medium to produce arachidonic acid or lipid incorporating arachidonic acid, and
recovering the arachidonic acid from the culture, or recovering the lipid from the culture and recovering arachidonic acid from the lipid, wherein
an additive which is a fat is added to the medium to increase the yield of arachidonic acid.

In a second aspect the invention provides a process for production of arachidonic acid, comprising
culturing a microorganism, of the genus Mortierella and capable of producing arachidonic acid, in a liquid medium comprising carbon source selected from glucose, fructose, xylose, saccharose, maltose, soluble starch, molasses, glycerol and mannitol, or on a solid medium selected from wheat bran, chaff or rice bran supplemented with water, to produce arachidonic acid or a lipid incorporating arachidonic acid, and
recovering the arachidonic acid from the culture, or recovering the lipid from the culture and recovering arachidonic acid from the lipid,
wherein
an additive which is octadecane, fatty acid, salt of fatty acid or a combination thereof is added to the medium to increase the yield of arachidonic acid.

In a third aspect the invention provides the use of a fat as an additive to the culture medium to increase the yield of arachidonic acid in a process comprising
culturing a microorganism, belonging to the genus Mortierella and capable of producing arachidonic acid, giving a culture product comprising arachidonic acid or a lipid of arachidonic acid, and
recovering the arachidonic acid or lipid thereof from the product.

A fourth aspect is the use of octadecane, fatty acid or salt thereof or combination of these as an additive to the culture medium to increase the yield of arachidonic acid in a process comprising
culturing a microorganism, belonging to the genus Mortierella and capable of producing arachidonic acid, in a culture medium which is a liquid medium containing carbon source selected from glucose, fructose, xylose, saccharose, maltose, soluble starch, molasses, glycerol and mannitol, or a solid medium selected from wheat bran, chaff or rice bran supplemented with water, giving a culture product comprising arachidonic acid or a lipid of arachidonic acid, and
recovering the arachidonic acid or lipid thereof from the product.

In the present invention, as a producer microorganism, any strain belonging to the genus Mortierella capable of producing arachidonic acid can be used. For example, Mortierella elongata IFO 8570, Mortierella exigua IFO 8571, and Mortierella hygrophila IFO 5941 can be used. These strains are stored in the Osaka Institute for Fermentation; 17-85, Juso-honmachi 2-chome, Yodogawa-ku, Osaka 532, Japan, and are available to the public without limitation.

Moreover, a new strain Mortierella elongata SAM 0219 can be used. This strain was newly isolated from a soil and identified by the present inventor, and was deposited with the Fermentation Research Institute, Agency of Industrial Science and Technology (FRI), Higashi 1-1-3, Yatabe-cho, Tsukuba-gun, Ibaraki-ken, Japan as FERM P-8703 on March 19, 1986, and transferred to International deposition under the Budapest Treaty as FERM BP-1239 on December 22, 1986.

The above-mentioned new strain SAM 0219 (FERM BP-1239) has the following taxonomical properties:

### Cultural characteristics on various culture media

### Culture condition: 25°C in the dark

### 1. Malt extract agar medium

Colonies growing fast, attaining a diameter of 28 to 31 mm in two days and a diameter of 65 to 72 mm in five days; colonies are lobed; the formation of aerial mycelium is scanty; sporulation is good; sporangiophores arising from the aerial hyphae; the mycelium has a garlic-like odour.

### 2. Potato dextrose agar medium

Colonies growing fast, attaining a diameter of 27 to 31 mm in two days and a diameter of 75 to 80 mm in five days; colonies form a rosette pattern of dense lobes; much aerial mycelium is formed at the center of the colony; the reverse side of the colony is yellowish white or yellow in colour; sporulation is poor; the mycelium has a rather strong garlic-like odour.

### 3. Czapek's agar medium

Colonies growing moderately fast, attaining a diameter of 22 to 24 mm in two days and a diameter of 50 to 53 mm in five days; the formation of aerial mycelium is scanty; occasionally, the aerial hyphae cling tightly to each other; sporulation is abundant; the mycelium has a garlic-like odour.

### 4. LCA agar medium (prepared according to Koichiro Miura and Mitsuyo Y. Kudo, "an agar-medium for aquatic Hyphomycetes" Transactions of the Mycological Society of Japan vol. 11, p 116 - 118, 1970)

Colonies growing fast, attaining a diameter of 27 - 29 mm in two days and a diameter of 64 to 66 mm in five days; colonies are lobed; the formation of aerial mycelium is scanty, except at the center of the colony; sporulation is good; sporangiophores arising from the aerial hyphae; the mycelium has a garlic-like odour.

### Microscopic Examination

Sporangiophore, mode of branching sporangiophore, sporangium, sporangiospore, etc., were microscopically observed from microscopic preparates and the colony per se from various media.

A sporangiophore tapers and has a length of 87.5 to 320 µm, a width of 3 to 7.5 µm at the root, and a width of 1.0 to 2.5 µm at the top. A sporangiophore often branches at the root. A sporangium is spherical in form, has a diameter of 15 to 30 µm, contains many ascospores therein, and has an unclear color after the detaching of the sporangiospore. A sporangiospore is elliptical or, rarely, renal in form, has a smooth surface, and a size of 7.5 to 12.5 × 5 to 7.5 µm. A relatively large number of chlamydospores are formed. Chlamydosphores are present separately or, rarely, linked in a chain form. Occasionally, several mycelia appear from the edge of the chlamydosphore. Chlamydosphore is elliptical or subspherical in form, and has a size of 12.5 to 30 × 7.5 to 15 µm, or a diameter of 12.5 to 15 µm. Zygospores are not observed.

### Physiological Properties

| Optical growth condition: | |
|---|---|
| pH | 6 to 9, |
| Temperature | 20°C to 30°C; |

| Range for growth: | |
|---|---|
| pH | 4 to 10, |
| Temperature | 5°C to 40°C. |

On the basis of the above-mentioned taxonomical properties, and according to J.A. von Arx, "The Genera of Fungi Sporulating in Pure Culture" 3rd ed., J. Cramer, 1981; and K.H. Domsch, W. Gams and T.H. Anderson, "Compendium of Soil Fingi", Academic Press, 1980, the strain SAM-0219 of the present invention is considered to be a fungus belonging to the genus Mortierella, because a sporangium is formed at a top of a sporangiophore, sporangium has no collumella, the sporangiospore has no appendage, and the mycelium has a garlic-like odour.

Therefore, the taxonomical properties of the strain of the present invention were compared with those of a known species of the genus Mortierella according to W. Gams, A key to the species of Mortierella, Persoonia 9: p 381 - 391, 1977. As a result, on the grounds that the colony is not velvety, the mycelum has a garlic-like odour, a sporangiophore has a length of 87.5 to 320 µm, and branches at only its lower part and does not branch racemosely, and a sporangium contains many sporangiospore therein, the strain in question was considered to fall under the genus Mortierella, subgenus Mortierella, section Hygrophila. The section Hygrophila includes 22 species. According to a comparison of the present strain with these 22 species, the present strain is similar to Mortierella zychae, M. elongatula, and M. elongata.

Therefore, the strain of the present invention was compared with the above-mentioned three strains, referring to K.H. Domsch, W. Gams, and T.-H. Anderson, "Compendium of Soil Fungi", Academic Press, 1980; W. Gams, Some New or Noteworthy Species of Mortierella"; Persoonia 9: 111 - 140, 1976; G. Linnemann, "Mortierella Coemans 1863"; H. Zyche and R. Siepmann, "Mucorales Eine Beschreibung Aller Gattungen und Arten dieser Pilzgruppe", p 155 - 241, J. Cramer, 1965. The present strain is clearly different from M. zychae in the length and width of the sporangiophore at the base, and the size of the sporangium. Moreover, the present strain is different from M. elongatula in the shape and size of the sporangiospore. The present strain is different from M. elongata in that sporangiophore is shorter, the chlamydosphore is ellipsoidal or subglobose in form, chlamydospores are rarely linked to each other in a chain form, and give rise to a small number of radiating hyphae. However, the present inventors concluded that such differences between the present strain and M. elongata are not sufficient to distinguish the present strain from M. elongata, and thus identified the strain of the present invention as Mortierella elongata, and designated it as strain SAM 0219.

Spores, mycelia, or a preculture is used as an inoculum for culturing the present strains. The medium used may be a liquid or solid medium. A liquid medium contains as a carbon source, for example, glucose, fructose, xylose, saccharose, maltose, soluble starch, molasses, glycerol, or mannitol. Nitrogen sources include organic substances such as peptones, yeast extract, meat extract, casamino acid, corn steep liquor, and inorganic substances such as sodium nitrate, ammonium nitrate, ammonium sulfate, and the like. If necessary, inorganic salts such as phosphate salts, magnesium sulfate, ferrous sulfate and cupric sulfate, and vitamins may be included in a medium. The concentration of these components is selected so that such components do not adversely affect the growth of the microorganism used. Practically, the concentration of carbon source is 0.1 to 30% by weight, preferably 1 to 10% by weight, reflective to the total weight of the medium. The concentration of the nitrogen source is 0.01 to 5% by weight, preferably 0.1 to 2% by weight, relative to the total weight of the medium.

To enhance the production of arachidonic acid, in addition to the above-mentioned medium components octadecane fatty acids or salts thereof, or fats are added to a medium in an amount of 0.01% to 20%. More than two kinds of the additives can be added. Octadecane is added to a medium preferably at the start of culturing, and fatty acids or salts thereof are added preferably at the start of and/or during culturing. When such an additive is used during culturing, it is added at one time, stepwise, or continuously.

The culturing temperature ranges 5°C to 40°C, preferably 20°C to 30°C. A pH value of the medium is 4 to 10, preferably 6 to 9.

Culturing is preferably carried out with aeration and/or agitation, with shaking in a liquid medium, or with standing, and is usually carried out for 2 to 10 days.

When culturing is carried out on a solid medium, the solid medium is composed of wheat bran, chaff or rice bran supplemented with water in an amount of 50 to 100% by weight relative to the wheat bran, chaff or rice bran.

If necessary, the medium is supplemented with a small amount of nitrogen source, inorganic salts, and/or minor nutrients.

Culturing is carried out at a temperature of 5°C to 40°C, preferably 20°C to 30°C, for 3 to 14 days.

During culturing, lipids containing arachidonic acid are intracellularly accumulated. When a liquid medium is used, arachidonic acid is recovered from the cultured cells by the following procedure.

After culturing, cultured cells are collected from the cultured broth by a conventional means such as filtration or centrifugation, the cells are washed with water, and preferably, the washed cells are dried. Drying is carried out by, for example, lyophilization or air-drying. The dried cells are treated with an organic solvent or a mixture thereof, preferably under a nitrogen stream, to extract a lipid containing arachidonic acid. The organic solvent or mixture thereof is, for example, ethers such as ethyl ether, hydrocarbons such as hexane, alcohols such as methanol or ethanol, halo-hydrocarbon such as chloroform or dichloromethan, petroleum ether, as well as a mixture of chloroform, methanol and water, or a combination of methanol and petroleum ether alternately used. By distilling off the solvent, a lipid containing concentrated arachidonic acid is obtained.

Alternatively, wet cells can be subjected to extraction. In such a case, a water-miscible solvent such as methanol or ethanol, or a water-miscible solvent comprising the water-miscible solvent and water or other organic solvent is used. The extraction procedure is the same as described for dried cells.

The lipid thus obtained contains arachidonic acid in the form of a lipid compound such as fat. Although the arachidonic acid can be isolated in the form of a free acid, it is preferably isolated in the form of an ester with a lower alcohol, for example, as methyl arachidonate. By converting arachidonic acid to such an ester, it is easily separated from other lipid components, and from other fatty acids formed during culturing, such as palmitic acid, oleic acid, linoleic acid and the like, which are also esterified at the same time as the arachidonic acid is esterified. To obtain methyl arachidonate, for example, the lipid prepared as described above is treated with a 5 to 10% hydrochloric acid solution in absolute methanol or a 10 to 50% BF₃ solution in methanol for 1 to 24 hours at a room temperature.

The mixture thus obtained is extracted with an organic solvent such as hexane, ethyl ether or ethyl acetate, to recover methyl arachidonate. Next, the extract is dried over anhydrous sodium acetate, and the solvent is distilled under a reduced pressure to obtain a residue mainly comprising a fatty acid mixture. The mixture contains, in addition to the target compound, methyl arachidonate, methyl palmitate, methyl stearate, methyl oleate and the like. From the mixture, methyl arachidonate is isolated by column chromatography, low temperature crystallization, a urea- adducting method, or a combination thereof.

The isolated methyl arachidonate is then hydrolyzed with an alkali and extracted with an organic solvent such as ethyl ether, ethyl acetate, or the like to obtain a free arachidonic acid.

Alternatively, arachidonic acid can be obtained, without conversion to methyl ester, by alkalolysis with, for example, 5% sodium hydroxide at a room temperature for 2 to 3 hours, followed by extraction of the fatty acids from the alkalolysis product and isolation of the target arachidonic acid.

### Examples

The present invention will now be further illustrated by, but is by no means limited to, the following examples.

### Example 1.

20 ml of a medium containing 2% glucose, 1% yeast extract, and 0.2% Tween 20, as well as an additive, i.e., 0.5% of octadecane or different kinds or sodium salt of fatty acid or lipid listed in the following Table 1 (pH 6.0)wascharged in each 100 ml-volume Erlenmeyer flask, and the flasks were autoclaved at 120°C for 20 minutes. Mortierella elongata SAM 0219 (FERM BP-1239) were inoculated the medium and then cultured for 5 days at 28°C with rotary shaking at 200 rpm. The cultured broths were separately filtrated to obtain cells. The cells were then completely washed with water and lyophilized to obtain dried cells. The cells were extracted with a mixture of chloroform, methanol, and water, according to Bligh and Dyer's one phase extraction method, to obtain a whole lipid. The lipid was treated with a mixture of methanol and hydrochloric acid (95:5) at 20°C for three hours to esterify the arachidonic acid. The reaction mixture was extracted with ethyl ether to obtain a mixture of fatty acid methyl esters.

The mixture was separated by column chromatography using octa decylsilane with elution by 95% acetonitrile solution to obtain fractions containing methyl arachidonate. After the fractions were combined, the solvent was distilled off on a rotary evaporator to obtain purified methyl arachidonate. The methyl arachidonate preparation thus obtained was compared with a commercially available authentic methyl arachidonate preparation, by gas chromatography, high performance liquid chromatography, and mass spectrometry. Both preparations showed the same results, revealing that the preparation prepared in this Example is methyl arachidonate.

The weight of the dried cells, amount of whole lipid, amount of whole fatty acid methyl ester, content of methyl arachidonate, and amount of methyl arachidonate per cultured broth are set forth for each additive.

**Table 1**

| Additive | Weight of dried cells | Amount of whole lipid | Amount of whole fatty acid methyl esters | Content of methyl arachidonate | Amount of methyl arachidonate per broth |
|---|---|---|---|---|---|
| | (mg) | (mg) | (mg) | (%) | (mg/ml) |
| Octadecane | 330 | 95 | 88 | 20 | 0.88 |
| Sodium oleate | 290 | 81 | 64 | 25 | 0.80 |
| Sodium linoleate | 300 | 96 | 83 | 19 | 0.79 |
| Olive oil | 430 | 130 | 113 | 24 | 1.36 |
| Corn oil | 420 | 118 | 97 | 23 | 1.12 |
| Coconut oil | 380 | 98 | 78 | 25 | 0.98 |
| No addition | 300 | 85 | 68 | 22 | 0.75 |

As seen from the Table 1, the addition of octadecane, salts of fatty acids and lipid increased the production of arachidonic acid by 10 to 80% relative to the no-addition control.

### Example 2.

20 ml of a medium containing 2% glucose and 1% yeast extract was charged in 100 ml-volume Erlenmeyer flasks, and the flasks were autoclaved at 120°C for 20 minutes. Mortierella elongata SAM 0219 (FERM BP-1239) was inoculated into the medium, and then incubated at 28°C for 4 days. After the addition of 100 mg of a different kind of sodium salt of fatty acid or lipid into each flask, incubation was continued at 28°C for an additional 2 days. The cultures were separately filtered to obtain cells. The cells were then subjected to extraction, hydrolysis, and methyl-esterification according to the same procedure as described in Example 1. The amount of methyl arachidonate per dried cells and per cultured broth was as set forth for each additive in Table 2.

**Table 2**

| Additive | Amount of methyl arachidonate | |
|---|---|---|
| | mg/g dried cells | mg/ml broth |
| Sodium oleate | 46 | 0.79 |
| Sodium linoleate | 47 | 0.80 |
| Sodium linolenate | 54 | 0.76 |
| Olive oil | 44 | 0.96 |
| Soybean oil | 53 | 1.12 |
| Linseed oil | 48 | 0.95 |
| No addition | 49 | 0.74 |

As seen from Table 2, the addition of salts of fatty acids and lipides increased the production of arachidonic acid by 10 to 60% relative to the no-addition control.

## Claims

1. A process for production of anichidonic acid, comprising
culturing a microorganism, of the genus Mortierella and capable of producing arachidonic acid, in a culture medium to produce arachidonic acid or lipid incorporating arachidonic acid, and
recovering the arachidonic acid from the culture, or recovering the lipid from the culture and recovering arachidonic acid from the lipid,
wherein
an additive which is a fat is added to the medium to increase the yield of arachidonic acid.

2. A process for production of arachidonic acid, comprising
culturing a microorganism, of the genus Mortierella and capable of producing arachidonic acid, in a liquid medium comprising carbon source selected from glucose, fructose, xylose, saccharose, maltose, soluble starch, molasses, glycerol and mannitol, or on a solid medium selected from wheat bran, chaff or rice bran supplemented with water, to produce arachidonic acid or a lipid incorporating arachidonic acid, and
recovering the arachidonic acid from the culture, or recovering the lipid from the culture and recovering arachidonic acid from the lipid,
wherein
an additive which is octadecane, fatty acid, salt of fatty acid or a combination thereof is added to the medium to increase the yield of arachidonic acid.

3. A process according to claim 1 or claim 2 wherein the additive is added to the medium at the start of culturing.

4. A process according to claim 1 or claim 2 wherein the fat, fatty acid or salt of fatty acid additive is added to the medium during culturing.

5. A process according to any one of claims 1 to 4 wherein the microorganism is selected from the group consisting of Mortierella elongata IFO 8570, Mortierella elongata SAM 0219 (FERM BP-1239), Mortierella exigua IFO 8571, and Mortierella hygrophila IFO 5941.

6. Use of a fat as an additive to the culture medium to increase the yield of arachidonic acid in a process comprising
culturing a microorganism, belonging to the genus Mortierella and capable of producing arachidonic acid, giving a culture product comprising arachidonic acid or a lipid of arachidonic acid, and
recovering the arachidonic acid or lipid thereof from the product.

7. Use of octadecane, fatty acid or salt thereof or combination of these as an additive to the culture medium to increase the yield of arachidonic acid in a process comprising
culturing a microorganism, belonging to the genus Mortierella and capable of producing arachidonic acid, in a culture medium which is a liquid medium containing carbon source selected from glucose, fructose, xylose, saccharose, maltose, soluble starch, molasses, glycerol and mannitol, or a solid medium selected from wheat bran, chaff or rice bran supplemented with water, giving a culture product comprising arachidonic acid or a lipid of arachidonic acid, and
recovering the arachidonic acid or lipid thereof from the product.

8. Use according to claim 6 or claim 7 in which the arachidonic acid is recovered from the culture product as an ester.

## Patentansprüche

1. Verfahren zur Herstellung von Arachidonsäure durch
Kultivierung eines zur Gattung Mortierella gehörenden und zur Herstellung von Arachidonsäure in einem Kulturmedium fähigen Mikroorganismus, um Arachidonsäure oder ein Arachidonsäure enthaltendes Lipid herzustellen, und
Rückgewinnung der Arachidonsäure aus der Kultur oder Rückgewinnung des Lipids aus der Kultur und Rückgewinnung der Arachidonsäure aus dem Lipid, worin ein Additiv, welches ein Fett ist, dem Medium zugesetzt wird, um die Ausbeute an Arachidonsäure zu erhöhen.

2. Verfahren zur Herstellung von Arachidonsäure durch
Kultivierung eines zur Gattung Mortierella gehörenden und zur Herstellung von Arachidonsäure fähigen Mikroorganismus in einem Flüssigmedium, enthaltend eine aus Glucose, Fructose, Xylose, Saccharose, Maltose, lösliche Stärke, Molasse, Glycerol und Mannitol ausgewählte Kohlenstoffquelle, oder einem mit Wasser ergänzten aus Weizenkleie, Häcksel oder Reiskleie ausgewähltem Festmedium, um Arachidonsäure oder ein Arachidonsäure enthaltendes Lipid herzustellen, und
Rückgewinnung der Arachidonsäure aus der Kultur oder Rückgewinnung des Lipids aus der Kultur und Rückgewinnung der Arachidonsäure aus dem Lipid, worin ein Additiv, welches Octadecan, eine Fettsäure, ein Salz der Fettsäure oder eine Kombination davon ist, dem Medium zugesetzt wird, um die Ausbeute an Arachidonsäure zu erhöhen.

3. Verfahren nach Anspruch 1 oder 2,
**dadurch gekennzeichnet, daß**
das Additiv dem Medium am Beginn der Kultivierung zugesetzt wird.

4. Verfahren nach Anspruch 1 oder 2,
**dadurch gekennzeichnet, daß**
das Fett-, Fettsäure- oder Fettsäuresalz-Additiv dem Medium während der Kultivierung zugesetzt wird.

5. Verfahren nach einem der Ansprüche 1 bis 4,
**dadurch gekennzeichnet, daß**
ein aus der Gruppe bestehend aus Mortierella elongata IFO 8570, Mortierella elongata SAM 0219 (FERM BP-1239), Morteriella exigua IFO 8571 und Mortierella hygrophila IFO 5941 ausgewählter Mikroorganismus eingesetzt wird.

6. Verwendung eines Fettes als ein Additiv zu dem Kulturmedium, um die Ausbeute an Arachidonsäure in einem Verfahren durch
Kultivierung eines zur Gattung Mortierella gehörenden und zur Herstellung von Arachidonsäure fähigen Mikroorganismus zu erhöhen, wobei ein eine Arachidonsäure oder ein Lipid der Arachidonsäure enthaltendes Kulturprodukt erhalten wird, und
Rückgewinnung der Arachidonsäure oder deren Lipids aus dem Produkt.

7. Verwendung eines Octadecans, einer Fettsäure oder ihres Salzes oder einer Kombination davon als ein Additiv zu dem Kulturmedium, um die Ausbeute an Arachidonsäure in einem Verfahren durch
Kultivierung eines zur Gattung Mortierella gehörenden und zur Herstellung von Arachidonsäure fähigen Mikroorganismus zu erhöhen, in einem Kulturmedium, welches ein Flüssigmedium, enthaltend eine aus Glucose, Fructose, Xylose, Saccharose, Maltose, lösliche Stärke, Molasse, Glycerol und Mannitol ausgewählte Kohlenstoffquelle ist, oder ein mit Wasser ergänztes aus Weizenkleie, Häcksel oder Reiskleie ausgewähltes Festmedium ist, wobei ein eine Arachidonsäure oder ein Lipid der Arachidonsäure enthaltendes Kulturprodukt erhalten wird, und
Rückgewinnung der Arachidonsäure oder deren Lipids aus dem Produkt.

8. Verwendung nach Anspruch 6 oder 7,
**dadurch gekennzeichnet, daß**
die Arachidonsäure als Ester aus dem Kulturmedium rückgewonnen wird.

## Revendications

1. Un procédé de production d'acide arachidonique comprenant
- la culture d'un micro-organisme appartenant au genre Mortierella et capable de produire l'acide arachidonique, dans un milieu de culture pour produire de l'acide arachidonique ou un lipide comprenant l'acide arachidonique et
- la récupération de l'acide arachidonique à partir de la culture, ou la récupération du lipide à partir de la culture et la récupération de l'acide arachidonique à partir du lipide,
dans lequel un additif qui est une matière grasse est ajouté au milieu pour augmenter le rendement en acide arachidonique.

2. Un procédé de fabrication de l'acide arachidonique comprenant
- la culture d'un micro-organisme du genre Morteriella et capable de produire de l'acide arachidonique, dans un milieu liquide comprenant une source de carbone choisi parmi glucose, fructose, xylose, saccharose, maltose, amidon soluble, mélasses, glycérol et mannitol, ou sur un milieu solide choisi parmi son de blé, paille ou son de riz additionné d'eau, pour produire de l'acide arachidonique ou un lipide comprenant de l'acide arachidonique, et
- la récupération de l'acide arachidonique à partir de la culture, ou bien la récupération du liquide de la culture et la récupération de l'acide arachidonique du lipide,
dans lequel un additif qui est l'octadécane, un acide gras, un sel d'acide gras ou une combinaison de ceux-ci est ajoutée au milieu pour augmenter le rendement en acide arachidonique.

3. Un procédé selon la revendication 1 ou 2, dans lequel l'additif est ajouté au milieu au moment de mise en culture.

4. Un procédé selon l'une des revendications 1 ou 2, dans lequel le corps gras, l'acide gras ou le sel d'acide gras est ajouté au milieu au cours de la culture.

5. Un procédé selon l'une quelconque des revendications 1 à 4, dans lequel le micro-organisme est choisi dans le groupe consistant en Mortierella elongata IFO 8570, Mortierella elongata SAM 0219 (FERM BP-1239), Mortierella exigua IFO 8571, et Mortierella hygrophila IFO 5941.

6. Utilisation d'un corps gras en tant qu'additif au milieu de culture pour augmenter le rendement en acide arachidonique dans un procédé comprenant
- la culture d'un micro-organisme appartenant au genre Mortierella et capable de donner de l'acide arachidonique, pour donner un produit de culture comprenant de l'acide arachidonique ou un lipide de l'acide arachidonique, et
- la récupération de l'acide arachidonique ou du lipide en dérivant à partir du produit.

7. Utilisation d'octadécane, d'un acide gras ou d'un de ses sels ou d'une combinaison de ceux-ci en tant qu'additif à un milieu de culture pour augmenter le rendement en acide arachidonique dans un procédé comprenant
- la culture d'un micro-organisme appartenant au genre Morteriella et capable de produire de l'acide arachidonique dans un milieu de culture qui est un milieu liquide contenant une source de carbone choisi parmi glucose, fructose, xylose, saccharose, maltose, amidon soluble, mélasses, glycérol et mannitol, ou bien un milieu solide parmi son de blé, paille ou son de riz additionné d'eau, en donnant un produit de culture comprenant de l'acide arachidonique ou un lipide de l'acide arachidonique et
- la récupération de l'acide arachidonique ou du lipide en dérivant à partir du produit.

8. Utilisation selon la revendication 6 ou 7 dans laquelle l'acide arachidonique est récupéré du produit de culture sous forme d'ester.
